# FASCICULE DE BREVET EUROPEEN

(11) **EP 1 127 560 B1**
(45) Date de publication et mention de la délivrance du brevet: **10.01.2018**
(21) Numéro de dépôt: 01400441.0
(22) Date de dépôt: 20.02.2001
(51) Int. Cl.: A61F 2/38

(54) **PROTHÈSE DU GENOU À CAVITÉS DANS LA PARTIE DE TROCHLÉE**
KNIEPROTHESE MIT MULDEN IM TROCHLEÄREN TEIL
KNEE PROSTHESIS WITH CAVITIES IN THE TROCHLEAR PART

(30) Priorité: 24.02.2000 FR 0002310
(43) Date de publication de la demande: 29.08.2001
(73) Titulaire: Aesculap AG, 78532 Tuttlingen (DE)
(72) Inventeur: BIEGUN, Jean-François, 52000 Chaumont (FR); MARCEAUX, Pascal, 52000 Chaumont (FR)
(74) Mandataire: Eidelsberg, Olivier Nathan

(56) Documents cités:
- EP-A- 0 627 203
- EP-A- 0 864 306
- WO-A-89/06947
- WO-A-94/26212
- US-A- 5 800 552

## Description

L'invention concerne une prothèse totale du genou du genre qui comporte une partie tibiale, comportant une plaque de support sensiblement horizontale fixée par des moyens d'ancrage appropriés au tibia ayant subi une résection, un ménisque, généralement en matériau plastique, qui est posé sur la plaque tibiale de manière fixe ou amovible et un composant fémoral qui comporte un ou deux condyles d'une part, et une partie de trochlée d'autre part.

La surface extérieure du ou des condyles de la partie fémorale est généralement de forme sphérique. En coupe dans un plan saggital, c'est-à-dire un plan parallèle à l'axe du tibia et/ou à l'axe du fémur en position plié du genou et à l'axe antéro-postérieur, les condyles ont la forme d'un arc de cercle qui s'étend angulairement sensiblement sur le domaine de flexion normal d'un genou, à savoir de 0 à 120 ou 130°. Cette surface sphérique du condyle coopère par glissement avec une surface supérieure correspondante du ménisque qui est de forme complémentaire de la surface sphérique des condyles.

On connaît déjà une prothèse de ce genre, par exemple de la demande internationale N° WO92/03108 de la Société British Technology Group. Ce type de prothèse de l'art antérieur présente l'inconvénient qu'il est préférable de poser cette prothèse en conservant le ligament croisé postérieur (LCP). En effet, dans les prothèses totales du genou de l'art antérieur, le composant fémoral peut poursuivre au-delà de 0° (c'est-à-dire au-delà de la verticale - tibia et fémur parallèles -, soit dans le domaine d'angles de rotation négatifs, c'est-à-dire des angles pour lesquels le tibia viendrait à se rapprocher du fémur non pas par l'intérieur comme c'est le cas habituellement lorsque l'on plie le genou mais par l'extérieur ce qui est anormal), et peut entraîner une dislocation du ménisque ou de la partie fémorale, dans le cas d'une pose sans conservation du ligament croisé postérieur.

On connait également une prothèse totale de ce genre du brevet européen N° 0.653.927 au nom de Walker, issu de la demande PCT WO94/26212. Une cavité y est formée dans le condyle pour permettre une congruence parfaite pour un angle de flexion nul (genou étendu). Il est préférable également que cette prothèse soit posée en conservant le ligament croisé postérieur, pour les mêmes raisons que celles mentionnées précédemment.

Quant à US-A-5.800.552 il décrit une coopération entre le ménisque et la partie fémorale pour bloquer en flexion pour des angles négatifs. Cependant le blocage n'est pas bien assuré, le bord antérieur étant peu surélevé et la cavité étant également formée dans les condyles et non pas dans la partie de trochlée.

Suivant l'invention, on pallie ces inconvénients en proposant une prothèse totale du selon la revendication 1 qui peut être posée aussi bien avec que sans le ligament croisé postérieur, et notamment sans le ligament croisé postérieur, sans que pour autant une rotation suivant les angles négatifs puissent avoir lieu au-delà par exemple de -5° ou -10°.

On y parvient suivant l'invention par une prothèse totale du telle que défini par la revendication 1 et comportant une partie fémorale et un ménisque, la partie fémorale étant constituée de deux condyles et d'une partie de trochlée, les condyles ayant chacun une surface extérieure de forme complémentaire d'une surface supérieure respective du ménisque pour assurer ainsi une congruence de ces surfaces sur au moins une partie du domaine normal de flexion, et il est prévu dans la partie de trochlée deux cavités et les emplacements relatifs, les dimensions et les formes de ladite au moins une cavité et du bord supérieur antérieur du ménisque étant tels que, en position de butée du bord supérieur antérieur du ménisque dans la cavité, la partie fémorale est bloquée en rotation de flexion pour des angles négatifs au delà d'un angle limite inférieur négatif.

En prévoyant ainsi les cavités formées dans la partie de trochlée du composant fémoral pouvant venir en butée du côté antérieur contre la partie de bord supérieur du ménisque pour un angle de flexion par exemple de -5°, le composant fémoral vient, par l'intermédiaire de la cavité formée dans la trochlée, se bloquer en butée contre le ménisque et ne peut plus poursuivre sa rotation dans les angles négatifs. Par conséquent, la prothèse totale du genou ne peut pas se fléchir dans le mauvais sens même si le ligament croisé postérieur n'est pas présent. En effet, dans l'art antérieur, c'est le ligament croisé postérieur qui empêche cette rotation dans les angles négatifs. Suivant l'invention, même si le ligament croisé postérieur n'est plus présent, cette rotation dans les angles négatifs est empêchée. Par exemple, l'angle limite inférieur négatif est compris entre 0° et - 10°, de préférence -5°. Par exemple, le bord supérieur comporte au moins une saillie de forme, en coupe sagittale, complémentaire de la cavité ou des cavités de sorte que cette ou ces saillies viennent buter contre la ou les cavités. Par exemple, la ou les saillies ont une hauteur, par rapport à la base plane du ménisque, supérieure à celle des bords supérieurs postérieurs.

Aux dessins donnés uniquement à titre d'exemple, il est représenté un mode de réalisation de l'invention. Aux dessins :
la figure 1 est une vue en coupe d'une prothèse du genou suivant l'invention, dans la position étendue du genou, c'est-à-dire pour un angle de flexion de 0°,
la figure 2 est une vue en coupe de la même prothèse qu'à la figure 1, mais pour un angle de flexion négatif maximum à savoir par exemple -5°,
la figure 3 est une vue en coupe pour une position fléchie du genou à savoir pour un angle de flexion de 60°,
la figure 4 est une vue en coupe dans le plan saggital de la partie fémorale de la prothèse totale du genou suivant l'invention, et
la figure 5 est une vue en perspective d'un insert ou ménisque tibial d'une prothèse totale du genou suivant l'invention.
la figure 6 est une vue en perspective éclatée d'une prothèse de genou suivant l'invention ; et
la figure 7 est une autre vue en perspective de la prothèse de la figure 6.

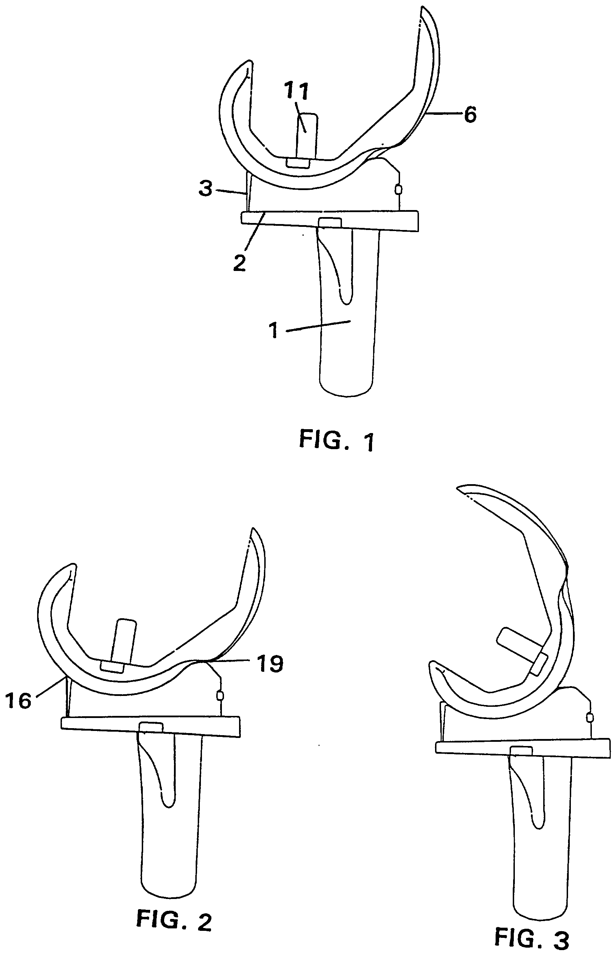

On définit l'angle de flexion par l'angle formé entre l'axe du tibia et l'axe du fémur dans le plan sagittal, par exemple le plan de la figure 1.

A la figure 1, l'angle de flexion est l'angle formé entre l'axe du plot 11 et l'axe 3 d'ancrage de la partie tibiale. A la figure 1, cet angle de flexion est nul. Pour une rotation du fémur par rapport au tibia fixe dans le sens inverse des aiguilles d'une montre, l'angle de flexion est positif. Par exemple, à la figure 3, cet angle est de +60°. Pour une rotation du fémur dans le sens des aiguilles d'une montre par rapport à un tibia fixe, l'angle de flexion est négatif, par exemple -5° à la figure 2.

A la figure 1, il est représenté une prothèse totale du genou en position étendue du genou. La prothèse totale du genou comporte une partie 1 tibiale constituée d'une plaque 2 tibiale issue d'un axe d'ancrage destiné à s'ancrer dans l'os du tibia. Sur la plaque 2 tibiale repose un insert 3 ou ménisque tibial en polyéthylène. Cet insert 3 tibial coopère avec la plaque 2 tibiale par l'intermédiaire d'un tenon 4 fixé à la plaque 2 tibiale et d'une cavité 5 formée dans l'insert tibial. L'insert tibial peut ainsi pivoter par rapport à l'axe du tenon 4 et se déplacer également en translations antéro-postérieur et médio-latéral dans un certain domaine de translations. On pourra se référer utilement aux demandes de brevet françaises N°9901158 déposée le 2 Février 1999 et N° 9908632 du 5 Juillet 1999 au nom de la demanderesse pour une description plus détaillée de cette partie de la prothèse totale du genou qui ne fait pas partie de la présente invention. La prothèse totale du genou comporte, en outre, une partie 6 fémorale.

Cette partie 6 fémorale comporte deux condyles 7 et 8 sphériques séparés par un intervalle dégagé intercondyles, et une partie de trochlée 20. En coupe dans le plan saggital, c'est-à-dire le plan parallèle à la fois à l'axe du tibia et à l'axe du fémur lorsque le genou est plié, les condyles 7 et 8 ont une forme circulaire. En fait, cette forme circulaire est constituée de deux segments circulaires côte-à-côte, un segment circulaire principal 9 et un segment circulaire d'extrémité 10. Le segment circulaire 10 d'extrémité s'étend sur un angle de environ 30° à 40° tandis que le cercle principal 9 s'étend sur un angle de 80° à 90°. Le rayon du cercle 10 secondaire est inférieur au rayon du cercle 9 principal. Le rayon du cercle 9 principal est égal à 1,25 fois le rayon du cercle 10 secondaire.

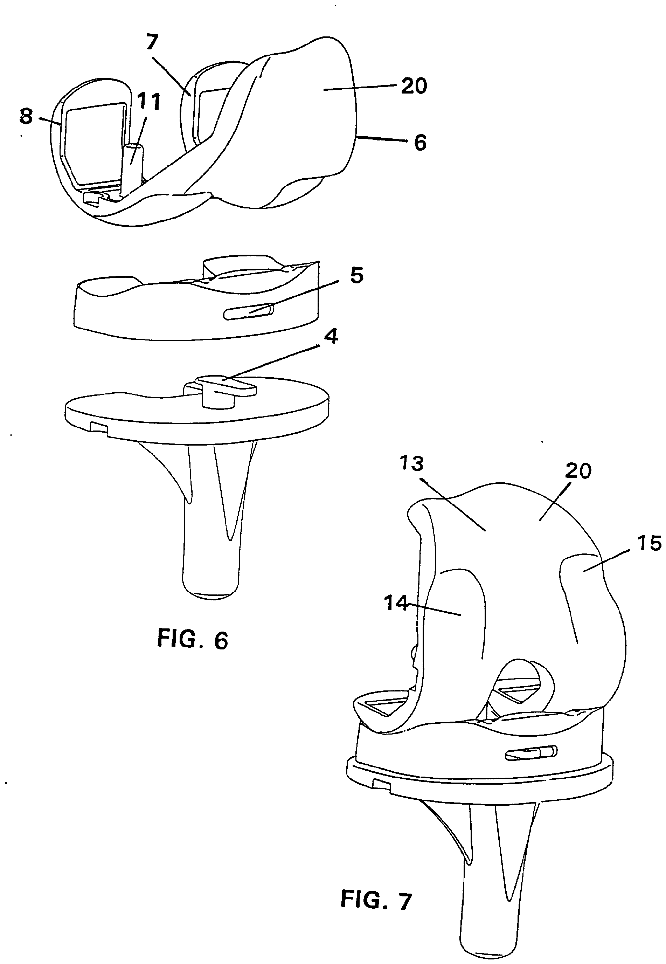

Deux plots (un seul étant vu à la figure 6) 11 assurent l'ancrage de la partie fémorale dans l'os du fémur. Ce plot 11 définit lorsqu'il est parallèle, comme c'est le cas à la figure 1 au tibia 1, l'angle de 0° qui correspond à une position étendue du genou.

Dans le domaine de flexion normal du genou, c'est-à-dire de 0° à 120° ou 130°, les condyles vont coopérer chacun avec une surface supérieure 12 de l'insert 3. Ces surfaces 12 ont, dans le plan saggital, une forme également circulaire de forme complémentaire de la forme des condyles 7 et 8 sphériques, et notamment ont un rayon égal au rayon du cercle 9 principal. On a ainsi une congruence parfaite jusqu'à une flexion d'environ 80°.

La surface extérieure de portée de la partie de trochlée 20 s'étend au-delà des condyles et de l'espace intercondyles. Il y est défini un trajet 13 trochléen en forme d'un canal comme on le voit au mieux à la figure 7. La forme de ce trajet 13 trochléen, toujours dans le plan saggital est également un cercle, notamment de rayon plus grand que le rayon du cercle 9 principal.

Deux cavités 14, 15 sont formées dans la surface extérieure de la partie de trochlée 20. Ces cavités 14, 15 sont formées par exemple par découpe.

L'insert 3 tibial ou ménisque tibial comporte deux bords 16, 17 postérieurs délimitant chacun du côté postérieur une des surfaces 12 supérieures en forme sphérique de l'insert 3. Du côté antérieur, l'insert 3 tibial est délimité par un bord 18. Ce bord 18 comporte deux parties de crête 19 qui, par rapport au plan défini par la base plane 21 de l'insert 3 tibial ont une hauteur supérieure à celles des bords 16, 17 postérieurs. Ces crêtes 19 qui sont ici incurvées circulairement sont, en coupe dans le plan sagittal, de forme complémentaire de celles des cavités 14, 15 de sorte que, pour une flexion du genou d'un angle négatif donné, c'est-à-dire une flexion du genou par exemple comme à la figure 2 de -5°, le composant fémoral va, lors de sa rotation en flexion, venir par ses cavités 14, 15 en butée contre le bord 18 et notamment au niveau des crêtes 19 de ce bord 18 et bloquer toute rotation au-delà de cet angle maximum négatif donné par exemple -5°, en direction des angles négatifs. Ainsi, il n'est plus nécessaire de prévoir le ligament croisé postérieur pour empêcher la flexion dans les angles négatifs non souhaitée. La forme des cavités 14, 15 peut être quelconque. L'important est qu'elle ait une forme complémentaire des crêtes 19 en coupe sagittale de l'insert 3 pour permettre une butée des cavités 14, 15 contre les crêtes 19.

A la figure 2, on voit la prothèse totale du genou en la position de flexion bloquée pour les angles négatifs, l'angle entre le tibia et le fémur étant ici de -5°.

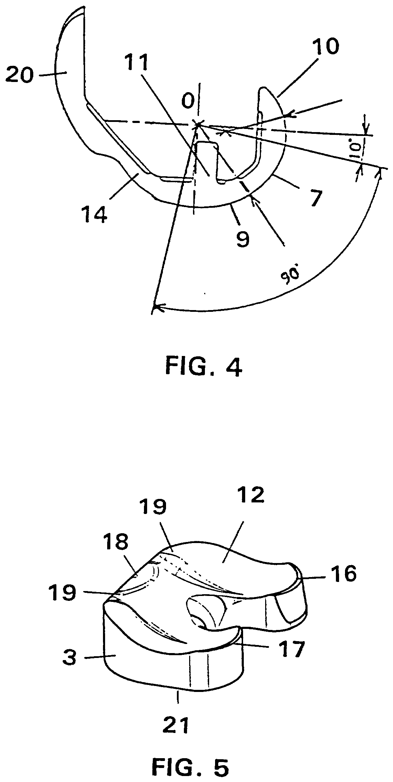

A la figure 4, il est représenté une vue en coupe sagittale du composant fémoral. Les cercles 9 et 10 correspondants aux condyles 7, 8, s'étendent sur un angle total de 130° et notamment 120° à partir du plot 11, dans le sens trigonométrique positif. Cet angle correspond au domaine normal de flexion, les condyles glissant sur le ménisque au fur et à mesure de la flexion du fémur par rapport au tibia.

La cavité 14 est formée dans la partie de trochlée à un niveau correspondant, à partir de la fin des condyles à un angle de 10° à 30° le long du cercle correspondant à la projection dans le plan sagittal de la partie de trochlée. La hauteur des crêtes du rebord 18 est telle qu'en position de butée des crêtes dans la cavité, l'angle de flexion est de -5°.

## Revendications

1. Prothèse totale du genou comportant une partie (6) fémorale et un ménisque (3), la partie fémorale (6) étant constituée de deux parties, à savoir une première ayant deux condyles (7, 8) séparés par un intervalle dégagé intercondyle et une deuxième partie dite de trochlée (20) dans laquelle l'intervalle dégagé intercondyle ne s'étend pas, les condyles ayant chacun une surface extérieure de forme complémentaire d'une surface (12) supérieure respective du ménisque (3) pour assurer ainsi une congruence de ces surfaces sur au moins une partie du domaine normal de flexion, la flexion étant définie par l'angle α formé entre l'axe du tibia et l'axe du fémur, un angle α = 0 correspondant au cas où la jambe est droite et les deux axes sont parallèles et un angle de flexion α positif correspondant à une flexion dans le sens normal de la flexion du genou dans laquelle le tibia tourne par rapport au fémur pour une flexion maximale d'environ 120° à 130°, tandis qu'un angle de flexion α négatif correspond à une flexion du genou dans laquelle le tibia tourne par rapport au fémur dans le sens opposé, **caractérisée par** deux cavités (14, 15) formées uniquement dans la partie de trochlée, les emplacements relatifs, les dimensions et les formes des cavités et d'un bord supérieur antérieur du ménisque étant tels que la partie fémorale peut fléchir en rotation jusqu'à un angle limite inférieur négatif et lorsque le bord (18) supérieur antérieur du ménisque vient en butée dans les cavités (14, 15), la partie fémorale est bloquée en rotation de flexion pour les angles négatifs au-delà de cet angle limite inférieur négatif les condyles étant constitués de deux segments circulaires en coupe sagittale, un segment (9) circulaire intermédiaire de plus grand rayon et un segment (10) circulaire d'extrémité de plus petit rayon, le segment circulaire intermédiaire (9) s'étendant sur un angle compris entre 80 et 90°, le segment circulaire d'extrémité (10) s'étend sur un angle d'environ 30° à 40°, chaque cavité étant formée dans la partie de trochlée à un niveau correspondant à un angle de 10 à 30° le long du segment de cercle qui correspond à la projection de la partie de trochlée dans le plan sagittal et qui prolonge le segment (9) circulaire intermédiaire du côté opposé au segment (10) circulaire d'extrémité.

## Patentansprüche

1. Knietotalprothese, umfassend einen Femurteil (6) und einen Meniskus (3), wobei der Femurteil (6) aus zwei Teilen besteht, nämlich einem ersten mit zwei Kondylen (7, 8), die durch ein freies interkondyläres Intervall getrennt sind, und einem zweiten Teil, der als trochleär (20) bezeichnet wird, in dem sich das freie interkondyläre Intervall nicht erstreckt, wobei die Kondylen jeweils eine Außenfläche mit einer komplementären Form zu einer jeweiligen oberen Fläche (12) des Meniskus (3) aufweisen, um so eine Kongruenz dieser Flächen über mindestens einen Teil des normalen Flexionsbereichs sicherzustellen, wobei die Flexion durch den Winkel α definiert wird, der zwischen der Achse der Tibia und der Achse des Femurs gebildet ist, wobei ein Winkel α = 0 dem Fall entspricht, wo das Bein gerade ist und die beiden Achsen parallel sind und ein positiver Flexionswinkel α einer Flexion in der normalen Flexionsrichtung des Knies entspricht, in der sich die Tibia in Bezug auf den Femur über eine maximale Flexion von ungefähr 120° bis 130° dreht, während ein negativer Flexionswinkel α einer Flexion des Knies entspricht, bei der sich die Tibia in Bezug auf den Femur in der entgegengesetzten Richtung dreht, **gekennzeichnet durch** zwei Mulden (14, 15), die nur in dem trochleären Teil gebildet sind, wobei die relativen Positionierungen, die Abmessungen und die Formen der Hohlräume und eines anterioren oberen Rands des Meniskus derart sind, dass sich der Femurteil in Rotation bis zu einem negativen unteren Grenzwinkel biegen kann, und wenn der anteriore obere Rand (18) des Meniskus in den Mulden (14, 15) in Anlage gerät, der Femurteil in Flexionsrotation für die negativen Winkel über diesen negativen unteren Grenzwinkel hinaus blockiert wird, wobei die Kondylen aus zwei kreisförmigen Segmenten im Sagittalschnitt bestehen, einem kreisförmigen Zwischensegment (9) mit größerem Radius und einem kreisförmigen Endsegment (10) mit kleinerem Radius, wobei sich das kreisförmige Zwischensegment (9) über einen Winkel zwischen 80 und 90° erstreckt, wobei sich das kreisförmige Endsegment (10) über einen Winkel von ungefähr 30° bis 40° erstreckt, wobei jede Mulde in dem trochleären Teil in einer Ebene gebildet ist, die einem Winkel von 10 bis 30° entlang des Kreissegments entspricht, welches der Projektion des trochleären Teils in der Sagittalebene entspricht und welches das kreisförmige Zwischensegment (9) auf der Seite gegenüber dem kreisförmigen Endsegment (10) verlängert.

## Claims

1. Complete knee prosthetic comprising a femoral part (6) and a meniscus (3), said femoral part (6) being in two parts, namely a first part comprising two condyles (7, 8) separated by an intercondyle clear interval and a said trochlean second part (20) in which said intercondyle clear interval does not extend, each condyle having a respective outer surface having a shape complementary to a respective upper surface (12) of said meniscus (3) to obtain a congruence of these surfaces on at least a part of the normal flexion range, said flexion being defined by the angle α defined between the tibia axis and the femur axis, an angle α = 0 corresponding to the case where the leg is straight and the two axis are parallel and a flexion angle α being positive for a knee flexion in the usual direction of knee flexion during which said tibia rotates relative to said femur for a maximum flexion of about 120° to 130°, while a flexion angle α being negative for a knee flexion during which said tibia rotates relative to said femur in the opposite direction, **characterized by** two cavities (14, 15) implemented only in said trochlea part, the relative positionings, the dimensions and the shapes of the cavities and of an anterior upper edge of the meniscus being such that said femoral part can flex in rotation up to an inferior negative limit angle and when said anterior upper edge (18) of said meniscus comes in abutment in said cavities (14, 15), said femoral part is blocked in rotation of flexion for the negative angles beyond said inferior negative limit angle, said condyles being made of two circular segments, in a sagittal section, that is an intermediary circular segment (9) of greater radius and an extremity circular segment (10) of smaller radius, said intermediary circular segment (9) extending itself along an angle of about 80° to 90°, said extremity circular segment (10) extending itself along an angle of about 30° to 40°, each cavity being formed in said trochlean part at a level corresponding to an angle of 10 to 30° along the circular segment corresponding to the projection of the trochlea part in the sagittal plane and which comes after said intermediary circular segment (9) on the side opposite said extremity circular segment (10).
